# EUROPÄISCHE PATENTSCHRIFT

(11) **EP 1 742 036 B1**
(45) Veröffentlichungstag und Bekanntmachung des Hinweises auf die Patenterteilung: **02.06.2010**
(21) Anmeldenummer: 06013841.9
(22) Anmeldetag: 03.07.2006
(51) Int. Cl.: G01N 17/00

(54) **Anordnung zum Prüfen von pharmazeutischen Substanzen**
System for testing pharmaceutical substances
Dispositif destiné à la vérification de substances pharmaceutiques

(30) Priorität: 04.07.2005 DE 202005010588 U
(43) Veröffentlichungstag der Anmeldung: 10.01.2007
(73) Patentinhaber: WEISS UMWELTTECHNIK GmbH, D-35447 Reiskirchen (DE)
(72) Erfinder: Hehl, Karl-Heinz, 35394 Giessen (DE)
(74) Vertreter: Stoffregen, Hans-Herbert

(56) Entgegenhaltungen:
- EP-A- 0 545 673
- WO-A-99/47963
- DE-A1- 4 330 759
- DE-A1- 10 117 723
- US-A- 5 487 872

## Beschreibung

Die Erfindung bezieht sich auf eine Anordnung zum Prüfen von Proben wie pharmazeutischen oder kosmetischen Substanzen umfassend einen einen Prüfraum umgebenden Schrank mit zumindest einer im Prüfraum angeordneten langgestreckten Bestrahlungsquelle wie Leuchtstoffröhre sowie zumindest eine Aufnahme wie Ablage für die Proben.

Entsprechende als Pharmaprüfschränke bezeichnete Anordnungen werden benötigt, um Aussagen über die pharmazeutischen Mittel bei deren Lagerung treffen zu können. Dabei sind nicht nur Temperatur und Klima, sondern insbesondere die Beleuchtung von Bedeutung. Um die Lichtbeeinflussung zu erfassen, ist es bekannt, Proben in einem Schrank in Fächern anzuordnen, in denen sich Tageslicht- und UV-Lampen befinden. Hierdurch ergeben sich Rückschlüsse über die Photostabilität.

Bei Prüfschränken, in denen parallel zur Ablage- bzw. Standfläche der Proben langgestreckte Bestrahlungszellen wie Leuchtstoffröhren zur Bestimmung der Lichtbeeinflussung der Proben angeordnet sind, ergibt sich der Nachteil, dass die Proben umgesetzt werden müssen, sofern die Proben über die Gesamtprüfzeit gleichen Lichtintensitäten ausgesetzt werden sollen; denn entsprechende langgestreckte Lichtquellen, insbesondere Leuchtstoffröhren, haben die Eigenschaft, über die Abstrahlungsoberfläche eine ungleichmäßige Abstrahlung zu zeigen, d.h. das Maximum liegt in der Mitte der Röhre und wird nach außen hin schwächer.

Aus dem Stand der Technik ist eine Vielzahl von Maßnahmen bekannt, um die Strahlung von Lichtquellen wie UV-Strahler oder Leuchtstofflampen zu beeinflussen. So sind nach dem DE-U-18 04 140 verstellbare Kunststofffilter vorgesehen, um eine gewünschte Strahlungsverteilung zu erreichen. Zur Veränderung der Intensitätsverteilung sind gesonderte Steuerungen vorgesehen. Die Strahlung kann auch durch Wärmeschutzfilter (DE-B-19 54 097), Streuscheiben (DE-A-197 14 234), Glasfilter (DE-A-43 30 759), optische Filter (JP-A-03-075 542) oder Reflektoren (DE-A-31 27 707) beeinflusst werden. Nach der EP-A-1 528 388 werden UV-Leuchtemissionsdioden verwendet, um gewünschte spektrale und räumliche Gegebenheiten bei der künstlichen Bewitterung von Proben zu erzielen. Nach dem DE-U-299 19 483 wird eine UV-Strahlung über hochreflektierende Reflektoren auf eine zu prüfende Probe geleitet, um gewünschte Leistung und Erhitzung der Probe zu erreichen.

Die DE-A-101 17 723 bezieht sich auf einen Probenträger für insbesondere biochemische Reaktionen. Zur Aufnahme von Testsubstanzen ist eine Lochplatte vorgesehen, die von einer Bodenplatte abgedeckt ist, über die konfokal-optische Messungen durchgeführt werden.

Die WO-A-99/47963 und die EP-A-0 545 673 beziehen sich auf Probenträger für biochemische Reaktionen bzw. Messeinrichtungen, um diese zu erfassen.

Gegenstand der US-A-5,487,872 ist ein für Ultraviolettstrahlung transparenter Probenträger.

Der vorliegenden Erfindung liegt die Aufgabe zugrunde, eine Anordnung der eingangs genannten Art so weiterzubilden, dass die die Proben beaufschlagende Strahlung vergleichmäßigt wird, d.h. die Lichtintensität über die gesamte Ablage- bzw. Stellfläche der Proben vergleichmäßigt ist, so dass ein Umsetzen der Proben nicht erforderlich ist.

Erfindungsgemäß wird das Problem im Wesentlichen dadurch gelöst, dass zwischen der zumindest einen Bestrahlungsquelle und der zumindest einen Aufnahme ein sich entlang der Bestrahlungsquelle erstreckendes auf die Aufnahme auftreffende Lichtintensität vergleichmäßigendes mechanisches mit Öffnungen in Form von Löchern oder Schlitzen versehenes Filter angeordnet ist. Bei dem mechanischen Filter handelt es sich insbesondere um ein Loch- oder Schlitzblech. Auch besteht die Möglichkeit, dass das mechanische Filter aus mehreren aneinander gereihten Elementen besteht, die zueinander beabstandet sind. Alternativ kann ein gerader Filter (gerades Blech) mit entsprechend dimensionierten Bohrungen oder Schlitzen eingesetzt werden.

Bevorzugterweise sieht die Erfindung vor, dass das mechanische Filter in Bezug auf die Aufnahme einen im Schnitt konkaven oder rinnenförmigen Geometrieverlauf zeigt. So kann das mechanische Filter im Querschnitt einen bogen- oder offenen trapezförmigen Verlauf aufweisen.

Insbesondere ist vorgesehen, dass in dem Prüfraum mehrere langgestreckte parallel zueinander verlaufende ein Leuchtfeld bildende Bestrahlungsquellen angeordnet sind und dass das mechanische Filter einen mittleren flächigen Abschnitt, der sich parallel zu dem Leuchtfeld erstreckt, sowie zwei von dem mittleren Abschnitt ausgehende in Richtung der Aufnahme abgewinkelte flächige Außenabschnitte aufweist.

Das mechanische Filter selbst besteht insbesondere aus Blechmaterial, wobei es sich um ein gerades oder gebogenes oder gefaltetes Blech handelt, das zur Erzeugung der gewünschten gleichmäßigen Intensitätsverteilung über die gesamte Probenaufnahmefläche entsprechende Durchbrechungen aufweist.

Durch die erfindungsgemäße Lehre wird das Strahlungsmaximum in der Mitte der Bestrahlungsquellen gedämpft und eine annähernd homogene Strahlung der Proben erreicht.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung eines der Zeichnung zu entnehmenden Ausführungsbeispiels.

Es zeigen:
- Fig. 1: eine Prinzipdarstellung eines Prüfraums eines Pharmaprüfschranks,
- Fig. 2: eine Draufsicht auf ein der Fig. 1 zu entnehmendes mechanisches Filter zur Intensitätsvergleichmäßigung und
- Fig. 3: eine Prinzipdarstellung eines Prüfschranks.

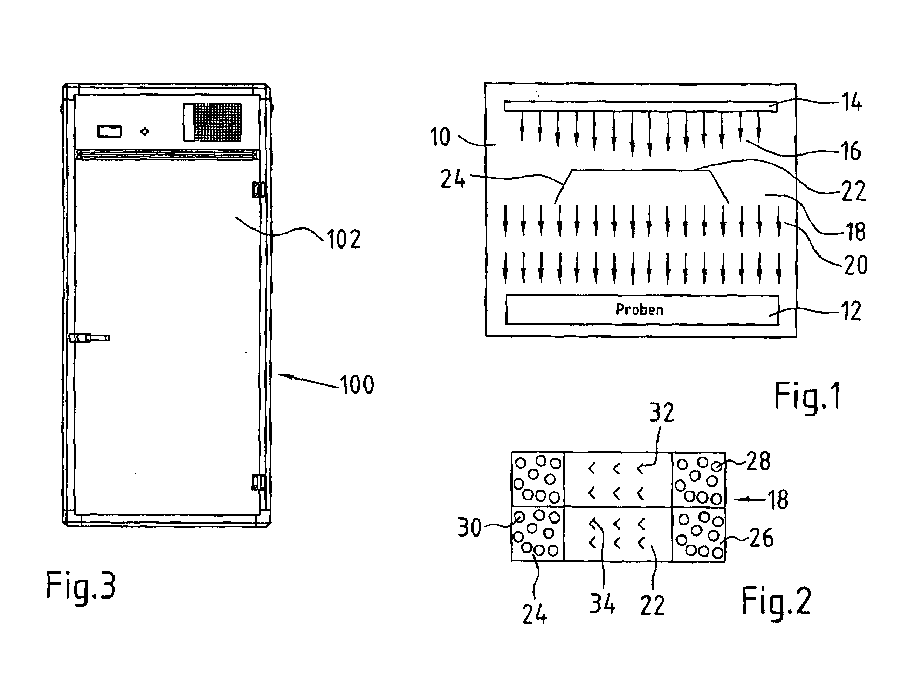

In Fig. 1 ist ein Prüfraum 10 eines Pharmaprüfschranks 100 dargestellt, in dem in einer Aufnahme bzw. auf einer Ablage 12 angeordnete Proben auf ihre Photostabilität hin überprüft werden sollen. Der Prüfraum 10 ist über eine den Prüfschrank 100 verschließbare Tür 102 zugänglich.

Oberhalb der Aufnahme 12 sind vorzugsweise mehrere ein Leuchtfeld bildende Bestrahlungsquellen 14 angeordnet. Hierbei handelt es sich insbesondere um Leuchtstoffröhren, von denen einige Tageslicht und andere UV-Licht emittieren. Hierzu können unterschiedliche Leuchtstoffröhren in der gewünschten Reihenfolge im Deckenbereich des Prüfraums 10 angeordnet sein. Die Bestückung kann gemischt (Tageslicht und UV-Licht) oder getrennt sein (pro Ebene nur Tageslicht oder nur UV-Licht).

Entsprechende langgestreckte Bestrahlungsquellen 14 wie Leuchtstoffröhren zeigen die Eigenschaft, dass entlang der Abstrahlfläche, also entlang ihrer Längsachse eine ungleichmäßige Abstrahlung derart erfolgt, dass ein Maximum in der Mitte der Bestrahlungsquelle 14 auftritt. Dies wird durch die unterschiedlich langen Pfeile 16, die von der Bestrahlungsquellen 14 ausgehen, symbolisiert.

Um ungeachtet dessen eine gleichmäßige Lichtintensitätsverteilung entlang der Aufnahme 12 für die Proben zu erzielen, ist zwischen der Aufnahme 12 und der Bestrahlungsquelle bzw. den Bestrahlungsquellen 14 ein mechanisches Filter 18 angeordnet, bei dem es sich insbesondere um ein gebogenes Lochblech handelt. Hierdurch wird erreicht, dass eine Vergleichmäßigung der Lichtintensität entlang der Aufnahme 12 erfolgt. Dies wird durch Pfeile 20 gleicher Längen symbolisiert.

Als Material für das Filter 18 kommt zum Beispiel auch Kunststoff in Frage.

Im Ausführungsbeispiel besteht das mechanische Filter 18 aus einem in Bezug auf die Aufnahme 12 offenes rinnenförmiges Element, das aus einem parallel zu den Bestrahlungsquellen 14 verlaufenden mittleren Abschnitt 22 und in Richtung der Aufnahme 12 abgewinkelte äußere Abschnitte 24, 26 besteht. Andere Geometrien wie eine bogenförmige Geometrie, die in Bezug auf die Aufnahme 12 einen konkaven Verlauf zeigt. Alternativ kann das Blech eine gerade Geometrie mit unterschiedlicher Lochung oder Schlitzung aufweisen, also in einer Ebene verlaufen und entsprechend der gewünschten Filterwirkung gelocht bzw. geschlitzt sein. Auch besteht die Möglichkeit, dass die Abschnitte 22, 24, 26 gelochte Bereiche (Löcher 28, 30) und/oder Schlitze 32, 34 aufweisen, um die gewünschte und über die gesamte Aufnahme der Probe 12 vergleichmäßigte Lichtintensität zu erreichen. Ein Aneinanderreihen der Abschnitte 22, 24, 26 zur Ausbildung des mechanischen Filters 18 ist gleichfalls möglich.

Durch die erfindungsgemäße Lehre ergibt sich der Vorteil, dass bei Photostabilitätstests für pharmazeutische Substanzen diese mit der notwendigen gleichmäßigen Intensität über der Probenabstellfläche ausgesetzt sind, so dass reproduzierbare Ergebnisse erzielbar sind, ohne dass es eines Umsetzens der Proben oder aufwändiger optischer Hilfsmittel bedarf.

Ist im Ausführungsbeispiel ein mechanisches Filter dargestellt, um die gewünschte Vergleichmäßigung der Intensität zu erzielen, besteht ohne Weiteres die Möglichkeit, dass mehrere Filter nebeneinander und/oder übereinander angeordnet werden. Das mechanische Filter kann Öffnungen in Form von Löchern oder Schlitzen aufweisen. Auch können nebeneinander angeordnete geschlossene Bleche verwendet werden, die zueinander beabstandet sind. Anstelle von Blechmaterialien können auch andere geeignete Materialien zur Ausbildung des mechanischen Filters benutzt werden.

## Patentansprüche

1. Anordnung zum Prüfen von Proben wie pharmazeutischen oder kosmetischen Substanzen umfassend einen einen Prüfraum (10) umgebenden Schrank (100) mit zumindest einer im Prüfraum angeordneten langgestreckten Strahlungsquelle (14) wie Leuchtstoffröhre sowie zumindest eine Aufnahme (12) wie Ablage für die Proben,
**dadurch gekennzeichnet,**
**dass** sich zwischen der Bestrahlungsquelle (14) und der zumindest einen Aufnahme (12) ein sich entlang der Bestrahlungsquelle erstreckendes die auf die Aufnahme auftreffende Lichtintensität vergleichmäßigendes mechanisches mit Öffnungen in Form von Löchern oder Schlitzen versehenes Filter (18) angeordnet ist.

2. Anordnung nach Anspruch 1,
**dadurch gekennzeichnet,**
**dass** das mechanische Filter (18) ein Loch- oder Schlitzblech ist.

3. Anordnung nach Anspruch 1 oder 2,
**dadurch gekennzeichnet,**
**dass** das mechanische Filter (18) in Bezug auf die Aufnahme (12) einen konkaven bzw. rinnenförmigen oder geraden Geometrieverlauf zeigt.

4. Anordnung nach zumindest einem der vorhergehenden Ansprüche,
**dadurch gekennzeichnet,**
**dass** das mechanische Filter (18) im Querschnitt eine bogen- oder offene trapezförmige Geometrie aufweist.

5. Anordnung nach zumindest einem der vorhergehenden Ansprüche,
**dadurch gekennzeichnet,**
**dass** in den Prüfraum (10) mehrere langgestreckte parallel zueinander verlaufende ein Leuchtfeld bildende Bestrahlungsquellen (14) angeordnet sind, dass das mechanische Filter (18) einen mittleren flächigen Abschnitt (22), der sich parallel zu dem Leuchtfeld erstreckt, sowie zwei in Richtung der Aufnahme (12) abgewinkelte flächige Außenabschnitte (24, 26) aufweist.

6. Anordnung nach zumindest einem der vorhergehenden Ansprüche,
**dadurch gekennzeichnet,**
**dass** das mechanische Filter (18) aus mehreren aneinander gereihten Elementen besteht.

7. Anordnung nach zumindest einem der vorhergehenden Ansprüche,
**dadurch gekennzeichnet,**
**dass** das mechanische Filter (18) aus Blechmaterial besteht.

8. Anordnung nach zumindest einem der vorhergehenden Ansprüche,
**dadurch gekennzeichnet,**
**dass** das mechanische Filter (18) ein gebogenes oder gefaltetes Blech oder ein Kunststoffteil ist.

9. Anordnung nach zumindest einem der vorhergehenden Ansprüche,
**dadurch gekennzeichnet,**
**dass** das mechanische Filter (18) eine im Schnitt einer Geraden folgende Geometrie aufweist.

10. Anordnung nach zumindest einem der vorhergehenden Ansprüche,
**dadurch gekennzeichnet,**
**dass** das mechanische Filter (18) eine gerade Ebene aufspannt.

## Claims

1. Arrangement for testing samples such as pharmaceutical or cosmetic substances comprising a cabinet (100) enclosing a testing room (10) with at least one elongated radiation source (14) such as a fluorescent tube arranged in the testing room as well as at least one receptacle (12) such as a storage place for the samples,
**characterized in**
**that** between the radiation source (14) and the at least one receptacle (12) a mechanical filter is arranged, that extends along the radiation source and homogenizes the light intensity impacting on the receptacle and is provided with openings in form of holes and slots.

2. Arrangement according to claim 1,
**characterized in**
**that** the mechanical filter (18) is a slotted or perforated plate.

3. Arrangement according to claim 1 or 2,
**characterized in**
**that** the mechanical filter (18) relative to the receptacle (12) shows a concave and channel-shaped course of geometry, respectively, or a straight course of geometry.

4. Arrangement according to at least one of the preceding claims,
**characterized in**
**that** in cross section the mechanical filter (18) has a bent or open trapezoidal geometry.

5. Arrangement according to at least one of the preceding claims,
**characterized in**
**that** in the testing room (10) are arranged several elongated radiation sources (14) running parallel to each other and forming a luminous field, and that the mechanical filter (18) comprises a middle plane section (22) extending in parallel to the luminous field, as well as two plane outer sections (24, 26) being angular towards the receptacle (12).

6. Arrangement according to at least one of the preceding claims,
**characterized in**
**that** the mechanical filter (18) consists of several elements strung together.

7. Arrangement according to at least one of the preceding claims,
**characterized in**
**that** the mechanical filter (18) consists of plate material.

8. Arrangement according to at least one of the preceding claims,
**characterized in**
**that** the mechanical filter (18) is a bent or folded plate or a plastic part.

9. Arrangement according to at least one of the preceding claims,
**characterized in**
**that** the mechanical filter (18) shows a geometry following in section a straight line.

10. Arrangement according to at least one of the preceding claims,
**characterized in**
**that** the mechanical filter (18) defines a plane surface.

## Revendications

1. Dispositif destiné à la vérification d'échantillons tels que des substances pharmaceutiques ou cosmétiques comprenant une armoire (100) renfermant une chambre d'essai (10) et présentant au moins une source de rayonnement allongée (14) disposée dans la chambre d'essai, telle qu'un tube fluorescent, et au moins un logement (12) tel qu'un support pour les échantillons,
**caractérisé en ce**
**qu'**est disposé, entre la source de rayonnement (14) et le logement (12) au moins en présence, un filtre mécanique (18), pourvu d'ouvertures en forme de trous ou de fentes, s'étendant le long de la source de rayonnement et homogénéisant l'intensité lumineuse incidente sur le logement.

2. Dispositif selon la revendication 1,
**caractérisé en ce**
**que** le filtre mécanique (18) est une tôle perforée ou fendue.

3. Dispositif selon la revendication 1 ou 2,
**caractérisé en ce**
**que** le filtre mécanique (18) présente par rapport au logement (12) un tracé géométrique concave ou en forme de goulotte ou droit.

4. Dispositif selon au moins l'une des revendications précédentes,
**caractérisé en ce**
**que** le filtre mécanique (18) présente en coupe une géométrie arquée ou trapézoïdale ouverte.

5. Dispositif selon au moins l'une des revendications précédentes,
**caractérisé en ce**
**que** plusieurs sources de rayonnement (14) allongées s'étendant parallèlement les unes par rapport aux autres et formant un champ lumineux sont disposées dans la chambre d'essai (10), que le filtre mécanique (18) présente une section centrale plane (22) qui s'étend parallèlement au champ lumineux, ainsi que de deux sections extérieures (24, 26) planes repliées en direction du logement (12).

6. Dispositif selon au moins l'une des revendications précédentes,
**caractérisé en ce**
**que** le filtre mécanique (18) est composé de plusieurs éléments alignés en rangs.

7. Dispositif selon au moins l'une des revendications précédentes,
**caractérisé en ce**
**que** le filtre mécanique (18) est composé de matériau en tôle.

8. Dispositif selon au moins l'une des revendications précédentes,
**caractérisé en ce**
**que** le filtre mécanique (18) est une tôle cintrée ou pliée ou une pièce en matière plastique.

9. Dispositif selon au moins l'une des revendications précédentes,
**caractérisé en ce**
**que** le filtre mécanique (18) présente en coupe une géométrie suivant une droite.

10. Dispositif selon au moins l'une des revendications précédentes,
**caractérisé en ce**
**que** le filtre mécanique (18) forme un plan droit.
